# EUROPÄISCHE PATENTSCHRIFT

(11) **EP 4 547 323 B1**
(45) Veröffentlichungstag und Bekanntmachung des Hinweises auf die Patenterteilung: **17.09.2025**
(21) Anmeldenummer: 24773374.4
(22) Anmeldetag: 17.09.2024
(51) Int. Cl.: A61N 1/36, A61F 9/00

(54) **STIMULATIONSELEKTRODENANORDNUNG FÜR EINE VORRICHTUNG ZUR ELEKTROSTIMULATION DES AUGES, VORRICHTUNG UND SYSTEM ZUR ELEKTROSTIMULATION DES AUGES, SET MIT EINER STIMULATIONSELEKTRODENANORDNUNG, SOWIE VERFAHREN**
STIMULATION ELECTRODE ASSEMBLY FOR A DEVICE FOR ELECTRICALLY STIMULATING THE EYE, DEVICE AND SYSTEM FOR ELECTRICALLY STIMULATING THE EYE, SET COMPRISING A STIMULATION ELECTRODE ASSEMBLY, AND METHOD
ENSEMBLE ÉLECTRODE DE STIMULATION POUR UN DISPOSITIF DE STIMULATION ÉLECTRIQUE DE L'¿IL, DISPOSITIF ET SYSTÈME DE STIMULATION ÉLECTRIQUE DE L'¿IL, ENSEMBLE COMPRENANT UN ENSEMBLE ÉLECTRODE DE STIMULATION, ET PROCÉDÉ

(30) Priorität: 18.09.2023 DE 102023125137
(43) Veröffentlichungstag der Anmeldung: 07.05.2025
(73) Patentinhaber: Okuvision GmbH, 72770 Reutlingen (DE)
(72) Erfinder: CERETTO GIANONE, Nicolò, 10129 Torino (TO) (IT); HENZLER, Paul, 72622 Nürtingen (DE)
(74) Vertreter: Witte, Weller & Partner Patentanwälte mbB
(86) Internationale Anmeldenummer: PCT/EP2024/075869
(87) Internationale Veröffentlichungsnummer: WO 2025/061662

(56) Entgegenhaltungen:
- WO-A1-2020/264263
- US-A1- 2020 171 307
- US-A1- 2020 391 029

## Beschreibung

Die vorliegende Erfindung betrifft eine Stimulationselektrodenanordnung für eine Vorrichtung zur Elektrostimulation des Auges, insbesondere zur transkornealen Elektrostimulation, wobei die Vorrichtung ein brillenartiges Gestell mit einem Nasenteil, eine mit dem Nasenteil verbundene Anordnung zum Halten des Gestells an dem Kopf des Patienten sowie zumindest einen an dem Gestellt angeordneten Elektrodenhalter für eine Stimulationselektrodenanordnung aufweist.

Die vorliegende Erfindung betrifft ferner eine entsprechende Vorrichtung zur Elektrostimulation des Auges, ein System mit Stimulationselektrodenanordnung und korrespondierender Vorrichtung, ein Set mit einer Stimulationselektrodenanordnung sowie ein Verfahren zum Befestigen einer Stimulationselektrodenanordnung an einer Vorrichtung zur Elektrostimulation des Auges.

Aus der WO 2011/086150 A2 sind Stimulationselektroden, Elektrodenhalter sowie Vorrichtungen zur Elektrostimulation grundsätzlich bekannt.

Die bekannte Stimulationselektrode ist eine drahtförmige Elektrode, die an dem brillenartigen Gestell befestigt wird. Wenn das Gestell an dem Kopf eines Patienten angeordnet wird, so gelangt die nachgiebige Drahtelektrode beim Aufsetzen des brillenartigen Gestells in Kontakt mit der vorderen Augenoberfläche im unteren Randbereich der Cornea des Auges. Eine Gegenelektrode ist entweder an dem Gestell vorgesehen oder wird gesondert am Patienten angebracht, beispielsweise an der Stirn des Kopfes oder an einem Ohrläppchen.

Über die Drahtelektrode und die Gegenelektrode kann ein pulsartiges elektrisches Stimulationssignal in das Auge geleitet werden, was dazu führt, dass ein Stimulationsstrom über die Cornea in das Auge fließt.

Die bekannte sowie andere Vorrichtungen werden eingesetzt, um elektrische Stimulationssignale in das Auge zu leiten, weil sich herausgestellt hat, dass dadurch bestimmte Formen der Retinopathia pigmentosa und andere Augenkrankheiten stabilisiert oder sogar verbessert werden können.

Netzhautdegeneration ist eine wesentliche Ursache für Erblindungen in den Industriestaaten. Verschiedene Untersuchungen geben Hinweise darauf, dass durch geringe elektrische Ströme, die durch die Netzhaut fließen, die erbliche, altersbedingte oder plötzlich auftretende Degeneration der Netzhaut verzögert werden kann.

Dies eröffnet die Möglichkeit, durch regelmäßige, also bspw. tägliche oder wöchentliche, elektrische Stimulation des Auges den allmählichen Verlust des Sehvermögens verzögern zu können, um so betroffenen Patienten länger Teile Ihres Augenlichtes zu erhalten oder sogar Teile des Augenlichtes wiederzugeben.

Diese Behandlung sollte zweckmäßigerweise vom Patienten selbst und zu Hause durchgeführt werden. Dabei muss im Extremfall angenommen werden, dass der Patient praktisch blind ist. Hieraus ergeben sich wesentliche Forderungen an die eingangs erwähnte Vorrichtung sowie die Stimulationselektrode.

Die drahtförmige Stimulationselektrode muss nämlich nach jeder Benutzung, zumindest aber sehr häufig ausgewechselt werden. Zum einen handelt es sich bei der drahtförmigen Stimulationselektrode um eine sehr dünne Elektrode, die bei unsachgemäßer oder auch nach häufiger Handhabung zerbrechen kann.

Zum anderen können es hygienische Erwägungen erforderlich machen, die Stimulationselektrode nach jeder Anwendung auszutauschen.

Die eingangs erwähnte WO 2011/086150 A2 beschreibt für diese Anwendungen drei verschiedene Ausführungsbeispiele.

In einem ersten Ausführungsbeispiel ist die Vorrichtung zur Elektrostimulation des Auges nach Art einer Brille ausgebildet, wobei an den Rahmen, die üblicherweise für die Gläser vorgesehen sind, dann spiralförmige Elektrodenhalter vorgesehen sind, die sich auf das Auge zu erstrecken. Für jedes Auge sind zwei derartige Elektrodenhalter vorgesehen, so dass eine Drahtelektrode mit den beiden inneren Enden dieser nachgiebigen Elektrodenhalter verbunden werden muss, um beim Aufsetzen der Brille dann in Kontakt mit der Cornea zu gelangen.

In einem zweiten Ausführungsbeispiel ist eine Gesichtsmaske gezeigt, die flächig an Teile des Gesichtes des Patienten angepasst ist. In dieser Gesichtsmaske sind Augenöffnungen vorgesehen, an denen Druckknöpfe vorgesehen sind, in die die drahtförmigen Stimulationselektroden eingehakt werden.

In einem dritten Ausführungsbeispiel ist eine Art "Schießbrille" gezeigt, bei der das brillenartige Gestell einen Nasenbügel und zwei davon abgehende Augenbügel umfasst, die wiederum mit klappbaren Seitenbügeln verbunden sind.

An den Augenbügeln sind jeweils zwei Elektrodenhalter befestigt, die längs des Augenbügels sowie quer zu dem Augenbügel, also in allen drei Richtungen verstellbar sind.

An ihren inneren freien Enden sind die Elektrodenhalter jeweils mit Ösen verbunden, in die die drahtförmige Stimulationselektrode eingelegt wird. Mit ihrem ersten Ende wird die Stimulationselektrode an dem nasenseitigen Elektrodenhalter befestigt und dann durch eine Öse in dem schläfenseitigen Elektrodenhalter geleitet. An ihrem zweiten Ende ist die Stimulationselektrode mit einem Gewichtskörper beschwert, so dass die Stimulationselektrode auf diese Weise vorgespannt wird.

Durch einen Fachmann wird dieses Gestell an den Kopf des Patienten angepasst, so dass sich beim Aufsetzen einer derartigen Brille, die mit einer oder zwei Stimulationselektroden bestückt ist, die Stimulationselektrode automatisch an das Auge anlegt.

Während das einmalige Einstellen der geometrischen Bedingungen des Gestells durch einen Fachmann erfolgt, soll der Patient anschließend die Vorrichtung zu Hause verwenden, um seine Augen regelmäßig elektrisch zu stimulieren.

Das dazu erforderliche Einhängen der Stimulationselektrode in die Ösen oder an die Elektrodenhalter gestaltet sich für Patienten mit Sehbehinderung jedoch extrem schwierig. Der dünne Elektrodenfaden wird teilweise von den Patienten nicht erkannt oder kann beim Aufsetzen des Brillengestells aus den Elektrodenhaltern herausgleiten.

Um dies zu verhindern, muss die Stimulationselektrode an dem nasenseitigen Elektrodenhalter mehrmals um den Halter gewickelt werden. Dies erfordert großes Geschick, weil der Faden zum einen starr ist und zum anderen die Patienten wegen ihrer Sehbehinderung auf ihr Tastgefühl angewiesen sind.

Vor diesem Hintergrund offenbart die DE 10 2011 055 844 B4 eine weiter verbesserte Stimulationselektrodenanordnung für eine Vorrichtung zur Elektrostimulation des Auges. Die Vorrichtung weist ein brillenartiges Gestell mit einem Nasenteil, eine mit dem Nasenteil verbundene Anordnung zum Halten des Gestells an dem Kopf des Patienten sowie zumindest einen justierbar an dem Nasenteil angeordneten Elektrodenhalter für die drahtförmige Stimulationselektrode auf. Die Stimulationselektrodenanordnung umfasst einen lösbar an dem Elektrodenhalter befestigbaren Elektrodenträger, an dem die Stimulationselektrode angeordnet ist. Der Elektrodenträger ist in einer Ausgestaltung als U-förmiger Bügel mit zwei Armen und einer nach außen offenen Nut ausgebildet. Mit der in der DE 10 2011 055 844 B4 beschriebenen Lösung wird eine Stimulationselektrodenanordnung der eingangs genannten Art bereitgestellt, welche einfach handzuhaben ist und auch sehbehinderten Patienten die Handhabung erleichtert.

Bei der in der DE 10 2011 055 844 B4 beschriebenen Lösung soll der Patient lediglich die Stimulationselektrodenanordnung an dem Elektrodenträger erfassen, der sehr viel massiver ist als die dünne Stimulationselektrode, und diesen Elektrodenträger in den bereits an der Vorrichtung vorhandenen und justierten Elektrodenhalter anbringen.

Weil der Elektrodenträger eine größere Masse und größere Abmaße aufweist als die Stimulationselektrode selbst, können auch sehbehinderte oder gar blinde Patienten den Elektrodenträger ertasten und lagerichtig in den Elektrodenhalter einsetzen.
Ferner offenbart WO 2020/264263 A1 Systeme und Schnittstellen für die Okulartherapie. US 2020/391029 A1 beschreibt eine tragbare medizinische Vorrichtung.
US 2020/171307 A1 beschreibt eine kopfgetragene Vorrichtung zur Therapie des Sehvermögens.

Vor diesem Hintergrund liegt der vorliegenden Erfindung die Aufgabe zugrunde, eine Stimulationselektrodenanordnung der eingangs genannten Art derart weiterzubilden, dass die Handhabung für sehbehinderte Nutzer nochmals weiter verbessert wird. Ferner wäre es wünschenswert eine Stimulationselektrodenanordnung der eingangs genannten Art bereitzustellen, welche flexibel bei unterschiedlichen Nutzern eingesetzt werden kann.

Die Erfindung ist in den beigefügten Ansprüchen definiert. Hier offenbarte Aspekte, Ausführungsformen und Beispiele, die nicht in den Umfang der beigefügten Ansprüche fallen, sind nicht Teil der Erfindung und werden lediglich zu Veranschaulichungszwecken bereitgestellt.

Gemäß einem ersten Aspekt der vorliegenden Offenbarung wird daher vorgeschlagen, eine Stimulationselektrodenanordnung bereitzustellen für eine Vorrichtung zur Elektrostimulation des Auges, wobei die Vorrichtung ein brillenartiges Gestell mit einem Nasenteil, eine mit dem Nasenteil verbundene Anordnung zum Halten des Gestells an dem Kopf des Patienten sowie zumindest einen (justierbar) an dem Gestell, insbesondere an dem Nasenteil, angeordneten Elektrodenhalter für eine (drahtförmige) Stimulationselektrode bzw. für eine Stimulationselektrodenanordnung mit einer (drahtförmigen) Stimulationselektrode aufweist, wobei die Stimulationselektrodenanordnung einen ersten Elektrodenträger und einen zweiten Elektrodenträger aufweist, wobei die Stimulationselektrode zwischen dem ersten Elektrodenträger und dem zweiten Elektrodenträger angeordnet ist, wobei der erste und/oder zweite Elektrodenträger dazu eingerichtet ist, magnetisch an dem Elektrodenhalter befestigt zu werden.

Die Erfinder haben erkannt, dass die in den eingangs genannten eigenen früheren Anmeldungen beschriebenen Lösungen die Handhabung einer Stimulationselektrode zur Elektrostimulation des Auges für sehbehinderte Nutzer bereits deutlich verbessern. Die Erfinder haben jedoch erkannt, dass das zielgenaue Platzieren der Stimulationselektrodenanordnung am Elektrodenhalter für sehbehinderte Nutzer weiterhin Schwierigkeiten bereitet.

Es wird daher vorgeschlagen, dass die Stimulationselektrodenanordnung einen ersten Elektrodenträger und einen zweiten Elektrodenträger aufweist, wobei die Stimulationselektrode zwischen dem ersten Elektrodenträger und dem zweiten Elektrodenträger angeordnet ist, wobei der erste und/oder zweite Elektrodenträger dazu eingerichtet ist, magnetisch an dem Elektrodenhalter befestigt zu werden. Indem der Elektrodenträger magnetisch an dem Elektrodenhalter befestigt wird, wird die Handhabung für sehbehinderter Nutzer weiter erleichtert. Elektrodenträger und Elektrodenhalter ziehen einander magnetisch an, sodass eine gewisse Selbstpositionierung von Elektrodenträger und Elektrodenhalter relativ zueinander erfolgen kann. Eine ungefähre bzw. nicht perfekte Positionierung des Elektrodenhalters, wie diese auch von einem sehbehinderten Nutzer erreicht werden kann, kann daher ausreichen, um den Elektrodenträger magnetisch aufzunehmen.

Ein weiterer Vorteil der vorgeschlagenen Lösung kann darin bestehen, dass die Hygiene bei der Handhabung einer Stimulationselektrodenanordnung für eine Vorrichtung zur Elektrostimulation des Auges verbessert werden kann. Die Erfinder haben erkannt, dass durch den magnetisch am Elektrodenhalter befestigbaren Elektrodenträger, weniger Versuche des sehbehinderten Nutzers zur korrekten Befestigung des Elektrodenträgers am Elektrodenträger erforderlich sein können. Hierdurch kann das Risiko, dass bei unsachgemäßer Handhabung Verunreinigungen in das Auge eingebracht werden, reduziert und die Hygiene bei der Handhabung weiter verbessert werden.

Ein weiterer Vorteil kann darin bestehen, dass die vorgeschlagene Stimulationselektrodenanordnung flexibel bei unterschiedlichen Nutzern eingesetzt werden kann. Bei der in der WO 2013079357 A1 bzw. dem erteilten deutschen Patent DE102011055844B4 aus dieser Patentfamilie beschriebenen Lösung wird ein einzelner Elektrodenträger mit einem u-förmigen Bügel vorgeschlagen. Ein solcher Bügel müsste jedoch bei Bedarf für den Nutzer angepasst werden bzw. müssten unterschiedliche Variationen für unterschiedliche Nutzer bereitgestellt werden. Dies erhöht die Variantenvielfalt und führt zu zusätzlichem Aufwand bei der Lagerhaltung.

Die Erfinder schlagen hingegen vor, einen ersten und einen zweiten Elektrodenträger, also insbesondere zwei separate Elektrodenträger bereitzustellen, zwischen denen die Stimulationselektrode angeordnet ist. Ein Vorteil kann darin bestehen, dass die beiden Elektrodenträger separat an einem oder mehreren Elektrodenhaltern befestigt werden können. Die Elektrodenhalter können hierbei vorjustiert an dem Gestell, insbesondere an dem Nasenteil, der Vorrichtung zur Elektrostimulation des Auges angeordnet sein. Vorzugsweise besteht abgesehen von der (flexiblen) Stimulationselektrode zwischen dem ersten und zweiten Elektrodenträger keine mechanische Verbindung, insbesondere kein Bügel mit fest vorgegebener Geometrie, zwischen dem ersten und zweiten Elektrodenträger. Die Elektrodenträger werden lediglich an diesen vorjustierten Elektrodenhaltern befestigt. Eine nutzerspezifische Anpassung der Stimulationselektrodenanordnung, d.h. eine Anpassung des bei jeder Verwendung auszutauschenden Elements, ist hingegen nicht mehr erforderlich. Hierdurch kann die Handhabung insbesondere für sehbehinderter Nutzer weiter verbessert werden. Ferner kann eine Variantenvielfalt und ein Aufwand bei der Lagerhaltung reduziert werden.

Ein weiterer Vorteil kann darin bestehen, dass die Stimulationselektrode sowie der erste und zweite Elektrodenträger als Stimulationselektrodenanordnung eine wirtschaftliche Einheit bilden, die vormontiert als Einmalartikel zur Verfügung gestellt werden kann.

Im Rahmen der vorliegenden Offenbarung kann unter einer Stimulationselektrodenanordnung eine Elektrodenanordnung zum Stimulieren und/oder Messen verstanden werden. Entsprechend kann unter einer Stimulationselektrode eine Elektrode zum Stimulieren und/oder Messen verstanden werden. Mit anderen Worten kann nicht nur ein elektrisches Stimulationssignal abgegeben werden, sondern auch ein Messsignal erfasst werden. Beispielsweise kann mit der Stimulationselektrode bzw. Stimulationselektrodenanordnung ein Stimulus appliziert werden oder ein Elektroretinogramm bzw. ERG-Signal erfasst werden. Entsprechend kann im Rahmen der vorliegenden Offenbarung unter einer Vorrichtung zur Elektrostimulation des Auges eine Vorrichtung zum Stimulieren und/oder Messen verstanden werden. Entsprechend kann im Rahmen der vorliegenden Offenbarung unter einem System zur Elektrostimulation des Auges ein System zum Stimulieren und/oder Messen verstanden werden.

Dabei ist es vorteilhaft, wenn der erste Elektrodenträger dazu eingerichtet ist, magnetisch an dem zumindest einen Elektrodenhalter befestigt zu werden und der zweite Elektrodenträger dazu eingerichtet ist, magnetisch an dem zumindest einen Elektrodenhalter befestigt zu werden. Der erste und zweite Elektrodenträger können an einem gemeinsamen Elektrodenhalter befestigt werden. Es können jedoch auch zwei Elektrodenhalter vorgesehen sein. Der erste Elektrodenträger kann dazu eingerichtet sein, magnetisch an einem ersten Elektrodenhalter befestigt zu werden. Der zweite Elektrodenträger kann dazu eingerichtet sein, magnetisch an einem zweiten Elektrodenhalter befestigt zu werden. Ein Vorteil dieser Ausgestaltung kann darin bestehen, dass der erste und zweite Elektrodenhalter jeweils wie gewünscht an einen Nutzer angepasst werden können. Insbesondere können der erste und zweite Elektrodenhalter dazu eingerichtet sein, separat voneinander justiert zu werden.

Der erste Elektrodenträger kann ein nasaler Elektrodenträger sein und dazu eingerichtet sein, magnetisch an einer nasalen Aufnahme des Elektrodenhalters befestigt zu werden. Der zweite Elektrodenträger kann ein temporaler Elektrodenträger sein und dazu eingerichtet sein, magnetisch an einer temporalen Aufnahme des Elektrodenhalters befestigt zu werden. Auch hierbei ist es möglich, dass zwei Elektrodenhalter vorgesehen sein können, ein erster, nasaler Elektrodenhalter und ein zweiter temporaler Elektrodenhalter. Der nasale Elektrodenhalter kann auch als nasenseitiger Elektrodenhalter bezeichnet werden. Der temporale Elektrodenhalter kann auch als schläfenseitiger Elektrodenhalter bezeichnet werden. Bei der Aufnahme kann es sich beispielsweise um einen Aufnahmestift handelt, auf welchen der Elektrodenträger, z.B. ein trichterförmiger Elektrodenträger, aufgesetzt wird.

In einer Ausgestaltung kann der erste und/oder zweite Elektrodenträger einen Magnet aufweisen und dazu eingerichtet sein, mittels des Magneten an dem Elektrodenhalter befestigt zu werden. Alternativ oder zusätzlich kann der mindestens eine Elektrodenhalter einen Magnet aufweisen und der Elektrodenträger dazu eingerichtet sein, mittels des Magneten an dem Elektrodenhalter befestigt zu werden. Beispielsweise können zwei Elektrodenhalter vorgesehen sein, die jeweils einen Magneten aufweisen. Ein Vorteil der Anordnung des oder der Magnete am Elektrodenhalter kann darin bestehen, dass die Magnete wiederholt bei mehreren Anwendungen wiederverwendet werden können. Es kann daher ausreichen, in den Elektrodenträgern der Stimulationselektrodenanordnung keinen Magneten, sondern lediglich ein (magnetisierbares) Gegenstück vorzusehen, welches vom Magneten im Elektrodenhalter angezogen wird. Hierdurch können die Kosten und der Materialeinsatz für die Stimulationselektrodenanordnung reduziert werden. Dies ist insbesondere deswegen vorteilhaft, da es hygienische Erwägungen erforderlich machen können, die Stimulationselektrode regelmäßig, insbesondere nach jeder Anwendung, auszutauschen.

Der erste und/oder zweite Elektrodenträger kann dazu ausgebildet sein, selbstausrichtend an dem Elektrodenhalter befestigt zu werden. Elektrodenhalter und Elektrodenhalter werden also magnetisch aneinander befestigt und richten sich hierbei ferner aneinander aus. Beispielsweise können Elektrodenhalter und Elektrodenträger selbstzentrierend ausgebildet sein. Ein Vorteil dieser Ausgestaltung kann darin bestehen, dass die Handhabung für einen sehbehinderten Nutzer weiter verbessert wird.

In einer Weiterbildung kann der erste und/oder zweite Elektrodenträger einen Trichter aufweisen und dazu eingerichtet ist, mittels des Trichters selbstausrichtend an dem Elektrodenhalter befestigt zu werden. Alternativ kann der erste und/oder zweite Elektrodenhalter einen Trichter aufweisen und der Elektrodenträger und Elektrodenhalter dazu eingerichtet sein, mittels des Trichters selbstausrichtend aneinander befestigt zu werden. Ein Vorteil dieser Ausgestaltung kann darin bestehen, dass die Handhabung für einen sehbehinderten Nutzer weiter verbessert wird. Eine sich verjüngende Trichterform erlaubt es dem sehbehinderten Nutzer, den Elektrodenträger und Elektrodenhalter grob in der Trichteröffnung zu positionieren und die Elemente dank der Führung durch den Trichter korrekt zu positionieren. Unter einem Trichter kann ein "Töpfchen" bzw. eine Aufnahme mit sich verjüngender Form verstanden werden. Der Trichter kann ferner dazu eingerichtet sein eine Verkippung von Elektroden und Elektrodenhalter zu vermeiden.

In einer Weiterbildung kann an einem Boden des Trichters ein elektrischer Kontakt angeordnet ist, welcher elektrisch leitend mit der Stimulationselektrode verbunden ist. Ein Vorteil dieser Ausgestaltung kann darin bestehen, dass mit dem Aufsetzen des Trichters eine elektrische Kontaktierung der Stimulationselektrode erfolgen kann.

In einer Weiterbildung kann ein magnetisierbares Metallelement am Boden des Trichters angeordnet sein. Das magnetisierbare Metallelement kann dazu eingerichtet sein, sowohl einen elektrischen Kontakt zur Stimulationselektrode bereitzustellen als auch magnetisch an dem Elektrodenhalter befestigt zu werden. Das magnetisierbare Metall kann einen elektrischen Kontakt, bereitstellen welcher elektrisch leitend mit der Stimulationselektrode verbunden ist. Es kann also ein Synergieeffekt erreicht werden, dass Befestigung und Stromübertragung der Stimulationselektrodenanordnung mit dem gleichen Element bereitgestellt werden können.

Der erste und/oder zweite Elektrodenträger kann dazu eingerichtet sein, in einer definierten Ausrichtung an dem mindesten einen Elektrodenhalter befestigt zu werden, insbesondere wobei der mindestens eine Elektrodenhalter eine ovale Aufnahme für den erste und/oder zweiten Elektrodenträger aufweist. Beispielsweise können ein erster und ein zweiter Elektrodenhalter vorgesehen sein, die jeweils eine ovale Aufnahme für den ersten und zweiten Elektrodenträger aufweisen. Der erste und zweie Elektrodenträger können beispielsweise einen mit der ovalen Aufnahme korrespondierenden ovalen Trichter aufweisen, welcher selbstzentrierend du auch selbstausrichtend daran befestigt werden können. Als Aufnahme kann der Elektrodenhalter beispielsweise einen Aufnahmestift aufweisen. Der Trichter des Elektrodenträgers kann auf einen solchen Aufnahmestift aufgestülpt werden. Dies erleichtert die Handhabung für einen sehbehinderten Nutzer. Unter oval kann im Rahmen der vorliegenden Offenbarung in einer ersten Richtung länger als in einer dazu orthogonalen zweiten Richtung breit zu verstehen sein.

Die Stimulationselektrode kann eine sogenannte DTL-Elektrode sein. Die Stimulationselektrode kann aus einem Draht aus Edelmetall oder einer Edelstahllegierung oder einem metallisch beschichteten Kunststofffaden gefertigt sein. Bei der DTL-Elektrode kann es sich um einen metallisch beschichteten Kunststofffaden, insbesondere um einen silberbeschichteter Nylonfaden, handeln.

Die Stimulationselektrode kann eine mit mindestens einem drahtförmigen Anschluss versehene Kontaktlinse mit Elektrodenstrukturen, insbesondere mit Elektrodenstrukturen aus einem Edelmetall, sein oder diese aufweisen. Ein Vorteil dieser Ausgestaltung kann in einer flächigeren Stimulation und einem verbesserten Eintrag des Stimulationssignals bestehen.

Die Stimulationselektrode kann mindestens an ihrem ersten Ende elektrisch leitend mit dem ersten Elektrodenträger verbunden sein. Der Elektrodenträger kann dazu eingerichtet sein, elektrisch leitend mit dem Elektrodenhalter verbunden zu werden. Bei dieser Maßnahme kann es von Vorteil sein, dass gleichzeitig mit dem mechanischen Befestigen des Elektrodenträgers an dem Elektrodenhalter auch die elektrische Kontaktierung der Stimulationselektrode erfolgt. Der Patient muss also keine weiteren Maßnahmen ergreifen, damit die mechanisch mithilfe des Elektrodenträgers an dem Elektrodenhalter befestigte Stimulationselektrode elektrisch kontaktiert werden kann. Die Stimulationselektrodenanordnung wird am Elektrodenhalter magnetisch gehalten, wobei mindestens einer der Elektrodenträger eine elektrische Verbindung mit der Aufnahme am Elektrodenhalter herstellt. Insbesondere kann der Elektrodenträger ein magnetisierbares Metall aufweisen und dazu eingerichtet sein, darüber sowohl magnetisch am Elektrodenhalter befestigt zu werden als auch elektrisch leitend mit dem Elektrodenhalter verbunden zu werden.

In einer Weiterbildung kann der erste Elektrodenträger mit einem elektrischen Steckkontakt für den Elektrodenhalter versehen sein und das erste Ende der Stimulationselektrode elektrisch mit dem Steckkontakt verbunden sein. Der elektrische Steckkontakt kann dazu eingerichtet sein, magnetisch am Elektrodenhalter befestigt zu werden. Eine elektrische Verbindung kann daher wie folgt bereitgestellt sein: Stimulationselektrode verbunden mit Metall am Boden des Trichters verbunden mit Magnet an Aufnahme des Elektrodenhalters verbunden mit Stimulationsquelle.

Die Stimulationselektrode kann durch zumindest eine Durchgangsöffnung an dem zweiten Elektrodenträger geführt und an ihrem zweiten Ende mit einem Gewichtskörper versehen sein. Durch die Verwendung von Gewichtskörpern wird das Anlegen einer neu eingesetzten Stimulationselektrode an das Auge derart vereinfacht, dass dies auch von einem stark sehbehinderten oder blinden Patienten vorgenommen werden kann. Weil der durch den Gewichtskörper belastete Elektrodenfaden durch eine Durchgangsöffnung an dem Elektrodenträger geführt ist, ist die Stimulationselektrode sozusagen nachgiebig gelagert, so dass sie sich problemlos an die Kontur des Auges anpasst.

Ferner betrifft die vorliegende Erfindung ein Set mit einer hierin offenbarten Stimulationselektrodenanordnung und einer Aufnahmeschale, wobei der erste Elektrodenträger und der zweite Elektrodenträger derart in der Aufnahmeschale angeordnet und dazu eingerichtet sind, dass sie mittels einer zwischen einem Elektrodenhalter und dem ersten und/oder zweiten Elektrodenträger wirkenden Magnetkraft aus der Aufnahmeschale entnehmbar sind. Der Nutzer muss also nicht mehr eine ggf. filigrane Stimulationselektrodenanordnung als solche handhaben, sondern kann stattdessen die Aufnahmeschale mit der darin angeordneten Stimulationselektrodenanordnung handhaben. Hierdurch kann die Handhabung insbesondere für sehbehinderter Nutzer weiter verbessert werden. Ein weiterer Vorteil kann darin bestehen, dass die Hygiene weiter verbessert werden kann, da die Stimulationselektrodenanordnung während der Anbringung nicht direkt angefasst werden muss.

Der erste Elektrodenträger und der zweite Elektrodenträger können mit einer Haltekraft klemmend in der Aufnahmeschale befestigt sein, wobei die Magnetkraft größer ist als die Haltekraft, so dass der ersten und/oder zweiten Elektrodenträger mittels der Magnetkraft aus der Aufnahmeschale entnehmbar sind. Mit anderen Worten kann die Stimulationselektrodenanordnung durch den ersten und/oder zweiten Elektrodenträger klemmend in einer Blisterpackung befestigt sein. Die Magnetkraft ist dabei stärker als die Haltekraft. Damit kann verhindert werden, dass die Elektrodenanordnung unbeabsichtigt rausfällt und verunreinigt wird. Die Hygiene kann damit weiter verbessert werden. Die Aufnahmeschale ist also derart eingerichtet, dass die Elektrodenträger so in der Aufnahmeschale durch eine Klemmkraft gehalten werden, dass sie bei geöffneter Aufnahmeschale nicht rausfallen können. Die Magnetkraft wird dann so gewählt, dass sie ausreicht, um die Elektrodenträger aus der Aufnahmeschale zu ziehen.

Der erste Elektrodenträger und/oder der zweite Elektrodenträger können mit einer Haltekraft klemmend jedoch (in mindestens einer Richtung) verkippbar in der Aufnahmeschale befestigt sein. Einer oder beide der Elektrodenträger können in der Aufnahmeschale leicht verkippt werden, ohne dass sie von den Klemmpunkten gelöst werden. Dadurch kann der Höhenunterschied bei Annähern der Elektrodenhalter ausgeglichen werden. Beispielsweise kann sich ein Höhenunterschied zwischen einem nasalen und einem temporalen Elektrodenhalter ergeben, wenn diese für unterschiedliche Nutzer in unterschiedlichen Positionen justiert sind. In einem solchen Fall kann es vorkommen, dass ein nasaler und ein temporaler Elektrodenhalter bei der Befestigung der Elektrodenträger and den jeweiligen Elektrodenhaltern nicht senkrecht auf die Elektrodenträger abgesenkt werden, sondern etwas verkippt, um einen Höhenunterschied auszugleichen.

Die Aufnahmeschale kann einen Deckel aufweisen. Insbesondere können Aufnahmeschale und der Deckel als Blisterpackung ausgebildet sein. Dies ermöglicht eine einfache Handhabung auch sehbehinderten Personen bei gleichzeitig verbesserter Hygiene.

Ferner betrifft die vorliegende Erfindung gemäß einem zweiten Aspekt eine Vorrichtung zur Elektrostimulation des Auges, die ein brillenartiges Gestell mit einem Nasenteil, eine mit dem Nasenteil verbundene Anordnung zum Halten des Gestells an dem Kopf eines Patienten sowie zumindest einen (justierbar) an dem Gestell angeordneten Elektrodenhalter zur Aufnahme einer Stimulationselektrodenanordnung aufweist, wobei als Stimulationselektrodenanordnung die neue Stimulationselektrodenanordnung eingesetzt wird.

Das Nasenteil kann dabei einen Nasenbügel und zwei Augenbügel aufweisen, an denen jeweils ein Elektrodenhalter befestigt wird.

Die Augenbügel sind dabei weiter vorzugsweise gegeneinander elektrisch isoliert und einzeln kontaktiert, so dass eine Therapie mit unterschiedlichen Stromstärken für die beiden Augen möglich ist.

Die Anordnung zum Halten des Gestells weist dabei in an sich bekannter Weise zwei an dem Nasenteil befestigte, vorzugsweise klappbar befestigte Seitenbügel auf. Die Anordnung zum Halten des Gestells kann auch breitenvariable, insbesondere federnd breitenvariable Seitenbügel aufweisen.

Allgemein ist es noch bevorzugt, wenn eine Gegenelektrode für die Stimulationselektrode vorgesehen ist.

Ferner betrifft die vorliegende Erfindung gemäß einem dritten Aspekt ein System zur Elektrostimulation des Auges mit einer Stimulationselektrodenanordnung gemäß dem ersten Aspekt und einer korrespondierenden Vorrichtung zur Elektrostimulation des Auges gemäß dem zweiten Aspekt. Die Stimulationselektrodenanordnung und die korrespondierende Vorrichtung können ausgestaltet sein wie vorstehend beschrieben oder im Rahmen der vorliegenden Offenbarung beispielhaft erläutert.

Ferner betrifft die vorliegende Erfindung gemäß einem vierten Aspekt ein Verfahren zum Befestigen einer Stimulationselektrodenanordnung an einer Vorrichtung zur Elektrostimulation des Auges, wobei das Verfahren folgende Schritte aufweist:
a) Bereitstellen einer Vorrichtung zur Elektrostimulation eines Auges und einer Stimulationselektrodenanordnung;
b) Platzieren des ersten und/oder zweiten Elektrodenträgers der Stimulationselektrodenanordnung und des Elektrodenhalters der Vorrichtung aneinander; und
c) Magnetisches Befestigen des ersten und/oder zweiten Elektrodenträgers der Stimulationselektrodenanordnung an dem Elektrodenhalter der Vorrichtung.

Mit anderen Worten kann in Schritt b) der Elektrodenhalter der Vorrichtung am ersten und/oder zweiten Elektrodenträger der Stimulationselektrodenanordnung platziert werden. Alternativ kann der erste und/oder zweite Elektrodenträger der Stimulationselektrodenanordnung am Elektrodenhalter der Vorrichtung platziert werden. Die jeweiligen Elemente werden also nahe aneinander platziert, was auch für einen Nutzer mit Sehbehinderung möglich ist, und die letztendliche Befestigung und Ausrichtung kann durch das magnetische Befestigen erfolgen.

Die für die Stimulationselektrodenanordnung gemäß dem ersten Aspekt beschriebenen weiteren optionalen Ausgestaltungen und Weiterbildungen gelten entsprechend für die weiteren Aspekte der vorliegenden Offenbarung, also insbesondere für das Set, die Vorrichtung und das System zur Elektrostimulation des Auges.

Weitere Vorteile ergeben sich aus der Beschreibung der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in den Zeichnungen dargestellt und werden in der nachfolgenden Beschreibung näher erläutert. Es zeigen:
- Fig. 1: eine schematische perspektivische Darstellung eines Systems zur Elektrostimulation des Auges mit der neuen Stimulationselektrodenanordnung;
- Fig. 2: eine schematische Darstellung von Elektrodenhalter und Stimulationselektrodenanordnung mit Stimulationselektrode sowie erstem und zweitem Elektrodenträger;
- Fig. 3: einen schematischen Ablauf zum Befestigen einer Stimulationselektrodenanordnung an einem Elektrodenhalter;
- Fig. 4: eine erste schematische perspektivische Darstellung einer Stimulationselektrodenanordnung;
- Fig. 5: eine zweite schematische perspektivische Darstellung einer Stimulationselektrodenanordnung;
- Fig. 6: eine dritte schematische perspektivische Darstellung einer Stimulationselektrodenanordnung;
- Fig. 7: eine schematische Schnittdarstellung einer Stimulationselektrodenanordnung, welche magnetisch an Elektrodenhaltern befestigt ist;
- Fig. 8: eine schematische perspektivische Darstellung eines Elektrodenhalters einer Vorrichtung zur Elektrostimulation des Auges,
- Fig. 9: eine schematische perspektivische Darstellung des Elektrodenhalters aus Fig. 8 mit einem Set mit einer in einer Aufnahmeschale angeordneten Stimulationselektrodenanordnung;
- Fig. 10: eine schematische perspektivische Darstellung des Elektrodenhalters aus Fig. 8 mit einer daran magnetisch befestigten Stimulationselektrodenanordnung aus dem Set aus Fig. 9;
- Fig. 11: eine perspektivische Darstellung, eine Draufsicht und eine Schnittdarstellung eines nasalen Elektrodenträgers;
- Fig. 12: eine perspektivische Darstellung, eine Draufsicht und eine Schnittdarstellung eines temporalen Elektrodenträgers;
- Fig. 13: ein Flussdiagramm eines Verfahrens zum Befestigen einer Stimulationselektrodenanordnung an einer Vorrichtung zur Elektrostimulation des Auges

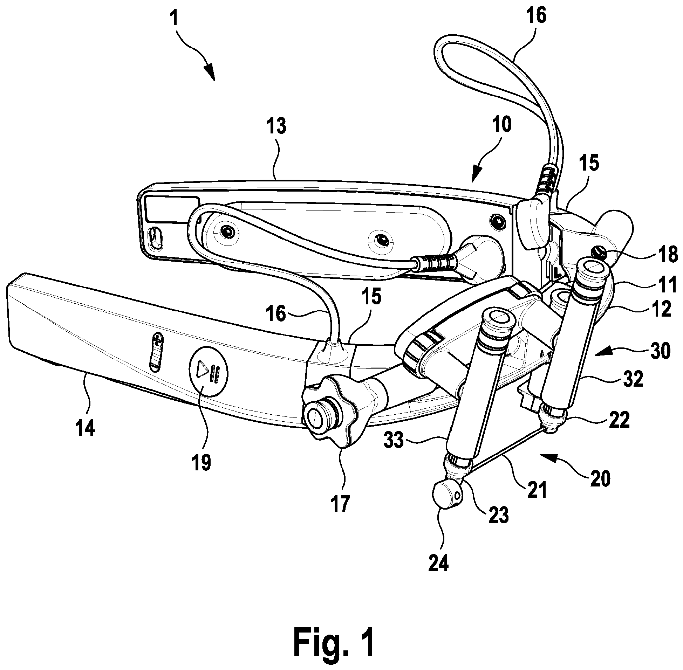

In Fig. 1 ist ein System 1 gezeigt, das zur Elektrostimulation des Auges eines Nutzers bzw. Patienten dient. Das System 1 weist eine Vorrichtung 10 zur Elektrostimulation des Auges sowie eine Stimulationselektrodenanordnung 20 auf.

Die Vorrichtung 10 umfasst ein brillenartiges Gestell 11 mit einem Nasenteil 12 und eine mit dem Nasenteil 12 verbundene Anordnung zum Halten des Gestells am Kopf des Patienten. Die Anordnung zum Halten des Gestells 11 am Kopf des Patienten kann zwei Seitenbügel 13, 14 aufweisen, wie bei einer Brille üblich. Optional können die Seitenbügel 13, 14 klappbar an dem Nasenteil 12 angelegt werden. Die Seitenbügel 13, 14 können auch über Federelemente 15 breitenvariabel mit dem Nasenteil 12 verbunden sein. Auf diese Weise ist es möglich, die Vorrichtung flexibel an den Kopf des Nutzers anzupassen.

Die Vorrichtung weist ferner einen an dem Gestell 11 angeordneten Elektrodenhalter 30 auf. Der Elektrodenhalter 30 kann hierbei vorzugsweise als schwenkbarer Elektrodenhalter ausgeführt sein. In diesem Fall kann der schwenkbare Elektrodenhalter zwischen einer angehobenen Position und einer definierten Behandlungsposition hin und her bewegt werden. In dem in Fig. 1 gezeigten Beispiel ist der Elektrodenhalter 30 als schwenkbarer Elektrodenhalter ausgeführt und befindet sich in einer angehobenen Position. Dies ermöglicht es einem Patienten, das brillenartige Gestell 11 mit angehobenem Elektrodenhalter 30 auf den Kopf aufzusetzen. Hierdurch wird die Handhabung erleichtert. In einem ersten Schritt wird lediglich das brillenartige Gestell aufgesetzt, es ist jedoch noch nicht erforderlich, dass auch die Stimulationselektrode 21 im gleichen Schritt auf das Auge aufgelegt wird.

Zur Elektrostimulation wird der Elektrodenhalter 30 abgesenkt und in die definierte Behandlungsposition gebracht, sodass die Stimulationselektrode 21 in Anlage mit der vorderen Augenoberfläche des Auges gelangen kann. Die Stimulationselektrode 21 wölbt sich dabei vorzugsweise so aus, dass sie sich passend an die Wölbung der Augenoberfläche, insbesondere der Cornea, anlegen kann. Dies ist möglich, weil die Stimulationselektrode 21 in sich flexibel und über den Gewichtskörper 24 nachgiebig in dem zweiten Elektrodenträger 23 gehalten sein kann.

In dem in Figur 1 gezeigten Beispiel kann der Elektrodenhalter beispielsweise über einen seitlich angeordneten Drehgriff 19 zwischen der angehobenen Position und einer definierten Behandlungsposition hin und her bewegt werden. Hierbei kann der Elektrodenhalter 30 drehbar bzw. schwenkbar am brillenartigen Gestell 11 gelagert sein. Beispielsweise mit einem Lager 18, wie für einen nicht abgebildeten Elektrodenhalter für ein linkes Auge gezeigt. Die definierte Behandlungsposition kann auch als Eingriffsposition bezeichnet werden. Die angehobene Position kann als Nicht-Eingriffsposition bezeichnet werden.

Für das rechte Auge und das linke Auge können separate Elektrodenhalter 30 vorgesehen sein. Zur Vereinfachung ist in Figur 1 nur ein Elektrodenhalter 30 dargestellt.

An dem Elektrodenhalter 30 ist die Stimulationselektrodenanordnung 20 magnetisch befestigt. Die Stimulationselektrodenanordnung 20 weist einen ersten Elektrodenträger 22 und einen zweiten Elektrodenträger 23 auf. Die Stimulationselektrode 21 ist zwischen dem ersten Elektrodenträger 22 und dem zweiten Elektrodenträger 23 angeordnet. Der erste und/oder zweite Elektrodenträger sind dazu eingerichtet, magnetisch an dem Elektrodenhalter 30 befestigt zu werden.

In dem in Figur 1 gezeigten Beispiel weist der Elektrodenhalter 30 einen ersten, nasalen Elektrodenhalter 32 und einen zweiten, temporalen Elektrodenhalter 33 auf. Der erste, nasale Elektrodenhalter 32 kann auch als nasenseitiger Elektrodenhalter bezeichnet werden. Der zweite, temporale Elektrodenhalter 33 kann auch als schläfenseitiger Elektrodenhalter bezeichnet werden. Der erste Elektrodenträger 22 ist ein nasaler Elektrodenträger und dazu eingerichtet, magnetisch an einer Aufnahme des nasalen Elektrodenhalters 32 befestigt zu werden. Der zweite Elektrodenträger 23 ist ein temporaler Elektrodenträger und dazu eingerichtet, magnetisch an einer Aufnahme des temporalen Elektrodenhalters 33 befestigt zu werden.

Die Stimulationselektrode 21 ist im montierten Zustand elektrisch leitend mit einem Stimulationsgerät zur Elektrostimulation des Auges verbunden. Vorzugsweise erfolgt die elektrische Verbindung in noch zu beschreibender Weise über den Elektrodenhalter 30. Das Stimulationsgerät erzeugt die gewünschten elektrischen Signale zur Elektrostimulation. Als Stimulationsgerät kann jedes beliebige Stimulationsgerät verwendet werden, das geeignete Stimulationssignale, insbesondere Sinuswellen oder pulsförmige elektrische Stimulationssignale für die Behandlung des Auges abgeben kann.

Das Stimulationsgerät kann in das brillenartige Gestell 11 integriert sein. Dies ist in Fig. 1 schematisch durch Bezugszeichen 19 und Bedienelemente zum Starten bzw. Pausieren der Elektrostimulation dargestellt. Die Stimulationseinrichtung kann jedoch auch als externe Stimulationseinrichtung vorgesehen sein.

Um den Stromkreis für die Elektrostimulation zu schließen können ferner Gegenelektroden 16 vorgesehen sein, welche beispielsweise an einer Stirn oder an einem Ohrläppchen des Patienten angebracht werden können.

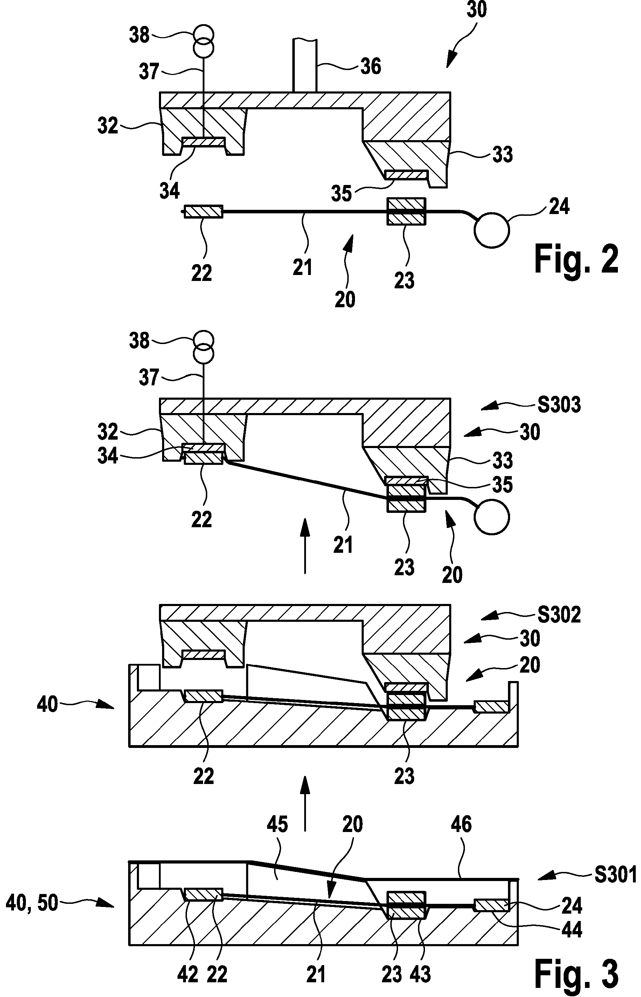

Fig. 2 zeigt eine schematische Darstellung von Elektrodenhalter 30 und Stimulationselektrodenanordnung 20. Die Stimulationselektrodenanordnung 20 weist den ersten Elektrodenträger 22 und den zweiten Elektrodenträger 23 sowie die dazwischen angeordnete Stimulationselektrode 21 auf. In dem gezeigten Beispiel ist die Stimulationselektrode 21 mit ihrem ersten Ende an dem ersten Elektrodenträger 22 befestigt und mit ihrem zweiten Ende durch den zweiten Elektrodenträger 23 hindurchgeführt und endet in einem Gewichtskörper 24.

Durch den Gewichtskörper 24 wird die Stimulationselektrode 21 im befestigten Zustand zwischen dem ersten Elektrodenträger 22 und dem zweiten Elektrodenträger 23 straff gespannt, kann aber wegen des geringen Gewichts des Gewichtskörpers 24 (bevorzugt zwischen 1 bis 20 g) leicht nach innen gebogen werden, wenn sie an die Cornea eines Auges angelegt wird.

Der erste und zweite Elektrodenträger 22, 23 und die Stimulationselektrode 21 sind, optional zusammen mit dem Gewichtskörper 24, zu einer Stimulationselektrodenanordnung 20 zusammengefügt, die austauschbar an dem Elektrodenhalter 30 befestigt werden kann.

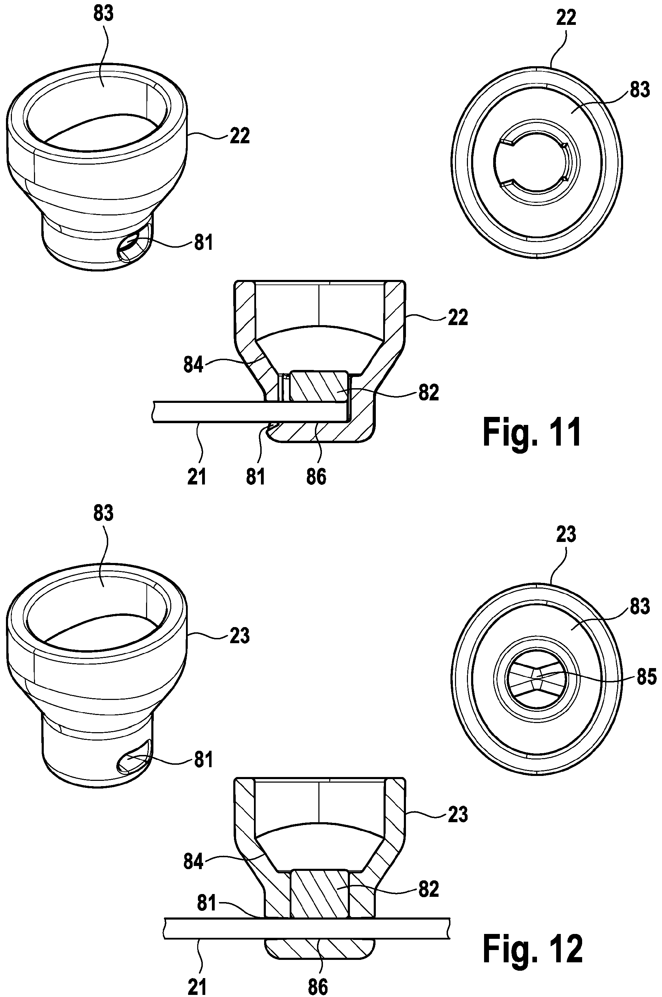

Der erste und/oder zweite Elektrodenträger 22, 23, vorzugweise beide, sind dazu eingerichtet magnetisch an dem Elektrodenhalter 30 befestigt zu werden. In dem in Fig. 2 gezeigten Beispiel weist der Elektrodenhalter 30 einen ersten, nasalen Elektrodenhalter 32 und einen zweiten, temporalen Elektrodenhalter 32 auf. Der erste Elektrodenhalter 32 weist hierbei einen ersten Magnet 34 auf und der erste Elektrodenträger 22 ist dazu eingerichtet durch den Magneten 34 am ersten Elektrodenhalter 32 befestigt zu werden. Entsprechend kann der zweite Elektrodenhalter 33 einen zweiten Magnet 35 aufweisen und der zweite Elektrodenträger 23 ist dazu eingerichtet, durch den Manget 35 am zweiten Elektrodenhalter 33 befestigt zu werden. Beispielsweise können der erste und/oder zweite Elektrodenträger ein magnetisierbares Metall aufweisen (siehe z.B. Element 82 in Fig. 11, Fig. 12), welches von den jeweiligen Magneten des ersten und zweiten Elektrodenhalters 32, 33 angezogen wird.

Wie in Fig. 2 schematisch dargestellt, kann ein Stimulationssignal zur Elektrostimulation über den Elektrodenhalter 30 bereitgestellt werden und der Stimulationselektrode 21 der Stimulationselektrodenanordnung 20 zugeführt werden. Eine elektrische Verbindung von einem Stimulationsgerät, schematisch mit Bezugszeichen 38 bezeichnet, kann über eine elektrische Verbindung 37 hergestellt werden. Im Rahmen der vorliegenden Offenbarung kann es sich bei dem Stimulationsgerät 38 um ein Gerät zum Stimulieren und/oder Messen handeln. Beispielsweise kann ein Stimulationssignal erzeugt werden, welches über die Stimulationselektrodenanordnung 20 mit der Stimulationselektrode 21 appliziert werden kann. Es kann sich jedoch auch beispielsweise um ein ERG-Gerät handeln, mit welchem ein Elektroretinogramm aufgezeichnet werden kann, welches mit der Stimulationselektrode 21 der Stimulationselektrodenordnung 20 erfasst wird.

Die Stimulationselektrode ist mindestens an ihrem ersten Ende elektrisch leitend mit dem ersten Elektrodenträger 22 verbunden ist, wobei der Elektrodenträger 22 dazu eingerichtet ist, elektrisch leitend mit dem Elektrodenhalter 32 verbunden zu werden. Vorzugsweise erfolgt die elektrische Verbindung elektrisch leitend über den Magneten 34, welcher den Elektrodenträger 22 am Elektrodenhalter 32 hält. Es ist jedoch auch denkbar, dass alternativ oder zusätzlich eine elektrische Verbindung über den zweiten Elektrodenträger 23 und den ersten Elektrodenhalter 33 hergestellt wird.

Es versteht sich, dass alternativ oder zusätzlich der erste Elektrodenträger 22 und/oder der zweite Elektrodenträger 23 jeweils einen Magnet aufweisen können und dazu eingerichtet sein können mittels des oder der Magneten an dem Elektrodenhalter 30 befestigt zu werden. Aus Kostengründen und um Ressourcen zu schonen ist es jedoch bevorzugt, wenn die austauschbare Stimulationselektrodenanordnung 20 keinen Magneten enthält. Sollte jedoch eine stabilere Befestigung erforderlich sein kann in einer Ausführungsform vorgesehen sein, dass auch die Stimulationselektrodenanordnung 20 einen oder mehrere Magnete zur magnetischen Befestigung aufweist.

In Fig. 2 weist der Elektrodenhalter 30 einen Befestigungsstift 36 auf, mit welchem der Elektrodenhalter an einem brillenartigen Gestell, wie beispielsweise aus der DE 10 2011 055 844 B4 bekannt, befestigt werden könnte. Dies ermöglicht eine vereinfachte Nachrüstung von herkömmlichen Systemen zur Elektrostimulation. Die vorgeschlagene Lösung wobei der erste und/oder zweite Elektrodenträger dazu eingerichtet ist, magnetisch an dem Elektrodenhalter 30 befestigt zu werden, kann vorteilhaft mit einem schwenkbaren Elektrodenhalter verbunden werden, ist jedoch nicht darauf beschränkt.

Fig. 3 zeigt einen schematischen Ablauf zum Befestigen einer Stimulationselektrodenanordnung 20 an einem Elektrodenhalter 30. Die Abfolge der Schritte S301, S302, S303 ist in Fig. 3 von unten nach oben dargestellt.

In einem ersten Schritt S301 wird eine Stimulationselektrodenanordnung 20 mit dem ersten Elektrodenträger 22 und dem zweiten Elektrodenträger 23, sowie der dazwischen anordneten Stimulationselektrode 21 und dem optionalen Gewichtskörper 24 bereitgestellt.

Statt nur die Stimulationselektrodenanordnung 20 also solche bereitzustellen, wird vorzugsweise ein Set 50 mit der Stimulationselektrodenanordnung 20 und einer Aufnahmeschale 40 bereitgestellt. Der erste Elektrodenträger 22 und der zweite Elektrodenträger 23 sind derart in der Aufnahmeschale 40 angeordnet und dazu eingerichtet sind, dass sie mittels einer zwischen einem Elektrodenhalter 30, 32, 33 und dem ersten und/oder zweiten Elektrodenträger 22, 23 wirkenden Magnetkraft aus der Aufnahmeschale 40 entnehmbar sind. Die Aufnahmeschalte 40 weist vorzugweise einen Deckel 46 auf. Die Stimulationselektrodenanordnung 20 kann damit in einem geschützten Raum 45 bereitgestellt werden. Die Aufnahmeschale 40 und der Deckel 46 können beispielsweise als Blisterpackung ausgebildet sein. Die Stimulationselektrodenanordnung 20 kann somit vor Verunreinigungen geschützt werden und auf einfache Art und Weise als Set vertrieben werden.

In dem gezeigten Beispiel weist die Aufnahmeschale 40 eine erste Halterung 42 für den ersten, nasalen Elektrodenträger 22, eine zweite Halterung 43 für den zweiten, temporalen Elektrodenträger und eine dritte Halterung 44 für den Gewichtskörper 24 auf. Die Elemente der Stimulationselektrodenanordnung 20 werden dank der Aufnahmeschale 40 an wohldefinierten Positionen bereitgestellt, welche an eine Geometrie des Elektrodenhalters 30 angepasst ist.

Bei den Halterungen 42, 43, 44 kann es ich um einfache Vertiefungen handeln, in welchen die korrespondierenden Elemente aufgenommen sind. Vorzugweise sind der erste Elektrodenträger 22 und der zweite Elektrodenträger 23 jeweils mit einer Haltekraft klemmend in der Aufnahmeschale 40 befestigt. Die Magnetkraft zwischen Elektrodenhalter 30 und dem ersten und zweiten Elektrodenträger ist dabei größer ist als die Haltekraft, so dass der erste und zweite Elektrodenträger 22, 23 mittels der Magnetkraft aus der Aufnahmeschale 40 entnehmbar sind.

Das Entnehmen der Stimulationselektrodenanordnung 20 aus der Aufnahmeschale 40 ist beispielhaft in Schritt S302 dargestellt. Hierbei wurde der Deckel 46 von Aufnahmeschale 40 entfernt. Die Stimulationselektrodenanordnung ist jetzt von der Oberseite her zugänglich. Der Elektrodenhalter 30 kann auf die sich noch in der Aufnahmeschale 40 befindliche Stimulationselektrodenanordnung 20 abgesenkt werden und diese magnetisch aufnehmen.

Hierdurch kann die Handhabung insbesondere für sehbehinderte Personen weiter vereinfacht werden. Bei der Aufnahmeschale 40 handelt es sich um ein geometrisch größeres Element als die eher fragile Stimulationselektrodenanordnung 20. Ein weiterer Vorteil kann darin bestehen, dass dank der magnetischen Aufnahme kein Einpressen der Stimulationselektrodenanordnung 20 in den Elektrodenhalter 30 erforderlich ist. Ein solches Einpressen könnte die Justage des Elektrodenhalters verändern und eine erneute Einstellung durch einen Fachmann erforderlich machen.

Die Aufnahmeschale 40 kann derart eingerichtet sein, dass der erste Elektrodenträger 22 gegenüber dem zweiten Elektrodenträger 23 erhöht in der Aufnahmeschale angeordnet ist. Dies kann die Handhabung erleichtern, da ein nasaler Elektrodenhalter 32 und ein temporaler Elektrodenhalter 33 oftmals unterschiedliche Abstände aufweisen. Die unterschiedlichen Abstände sind in der Schnittdarstellung in Figur 3 als unterschiedliche Höhen dargestellt. Alternativ oder zusätzlich sind der erste Elektrodenträger 22 und/oder der zweite Elektrodenträger 23 mit einer Haltekraft klemmend jedoch in mindestens einer Richtung verkippbar in der Aufnahmeschale 40 befestigt sind. Hierdurch können einerseits Höhenunterschiede bzw. unterschiedliche Augenabstände bei der Justage von nasalem und temporalem Elektrodenhalter 32, 33 ausgeglichen werden. Ferner kann eine unpräzise Platzierung des Sets 50 mit der Aufnahmeschale 40 relativ zum Elektrodenhalter 30 kompensiert werden.

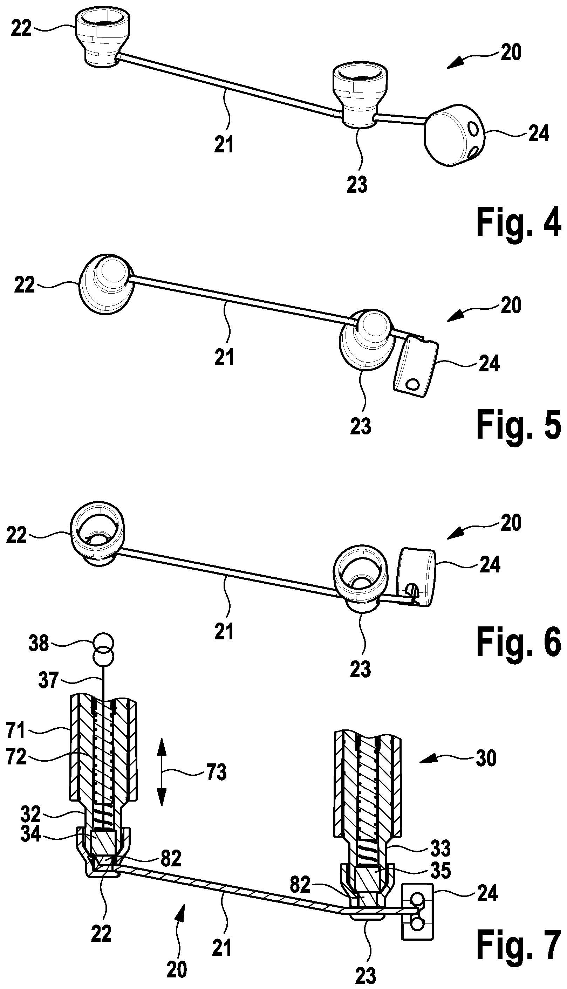

Fig. 4 bis Fig. 6 zeigen schematische perspektivische Darstellungen einer Stimulationselektrodenanordnung 20, welche dazu eingerichtet ist, magnetisch an einem Elektrodenhalter 30 befestigt zu werden.

Fig. 7 zeigt eine Schnittdarstellung, wobei die Stimulationselektrodenanordnung 20 an einem Elektrodenhalter 30 mit einem nasalen ersten Elektrodenhalter 32 und einem temporalen zweiten Elektrodenhalter 33 angebracht ist. Die Stimulationselektrodenanordnung 20 weist den ersten Elektrodenträger 22 und den zweiten Elektrodenträger 23 auf, wobei die Stimulationselektrode 21 zwischen dem ersten Elektrodenträger 22 und dem zweiten Elektrodenträger 23 angeordnet ist. Ferner weist die Stimulationselektrodenanordnung den optionalen Gewichtskörper 24 auf, wie vorstehend beschrieben.

Der erste Elektrodenträger 22, im vorliegenden Beispiel der nasale Elektrodenträger, ist in Fig. 11 mit einer perspektivischen Darstellung (links oben in Fig. 11), einer Draufsicht (rechts oben in Fig.11) und einer Schnittdarstellung (unten in Fig. 11) dargestellt.

Der zweite Elektrodenträger 32, im vorliegenden Beispiel der nasale Elektrodenträger, ist in Fig. 12 mit einer perspektivischen Darstellung (links oben in Fig. 12), einer Draufsicht (rechts oben in Fig.12) und einer Schnittdarstellung (unten in Fig. 12) dargestellt.

Um die Anbringung der Stimulationselektrodenanordnung 20 weiter zu erleichtern sind der erste und/oder zweite Elektrodenträger 22,23 dazu ausgebildet, selbstausrichtend an dem Elektrodenhalter 30 befestigt zu werden.

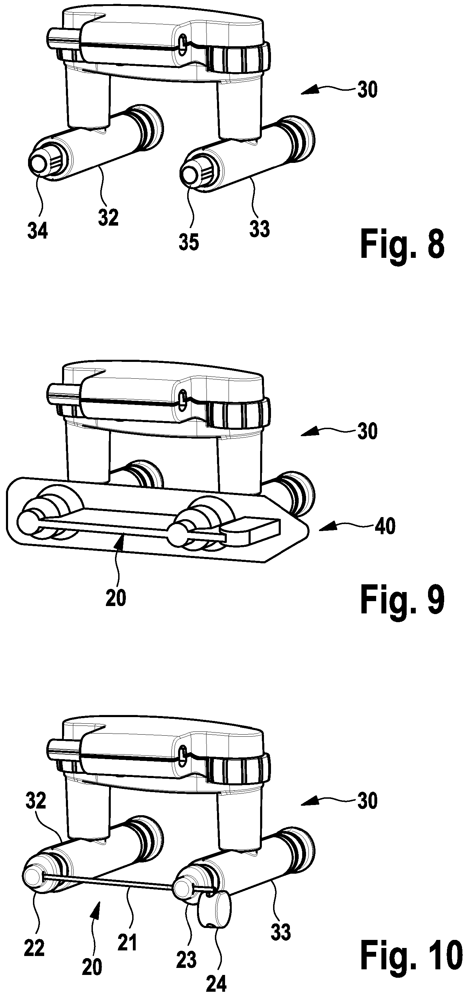

Wie in den Schnittdarstellungen in Figur 11 und Figur 12 gezeigt können der erste, nasale und zweite, temporale Elektrodenträger jeweils einen Trichter 84 aufweisen. Der Elektrodenhalter 30 kann, wie beispielsweise in Figur 7 und Figur 8 dargestellt, einen korrespondierende nasalen Elektrodenhalter 32 und temporalen Elektrodenhalter 33 aufweisen. Die Trichter 84 des nasalen und temporalen Elektrodenträgers 22, 23 können auf die entsprechenden stiftförmigen Aufnahmen des nasalen und temporalen Elektrodenhalters 32,33 aufgesetzt werden.

Wie in den Draufsichten in Fig. 11 und Fig. 12 gezeigt, weisen der erste und zweite Elektrodenträger 22, 23 eine ovale Aufnahme 83 für den ersten und zweiten Elektrodenhalter auf. Die Aufnahme 83 ist länger als breit, d.h. oval im Sinne der vorliegenden Offenbarung. Entsprechend weisen der erste und zweite Elektrodenhalter 32, 33, wie beispielsweise in Fig. 8 gezeigt, korrespondierende Aufnahmestifte mit ovaler Form auf. Es sind jedoch auch andere Ausgestaltungen denkbar, wobei der erste und/oder zweite Elektrodenträger 22, 23 dazu eingerichtet ist, in einer definierten Ausrichtung am Elektrodenhalter 30, 32, 33 befestigt zu werden.

Wie in Fig. 11 und Fig. 12 gezeigt, weisen der erste und zweite Elektrodenträger eine Öffnung 81 auf, in welche die Stimulationselektrode 21 eingeführt ist. Wie in Figur 12 in der unteren Darstellung gezeigt kann es sich bei dem zweiten, temporalen Elektrodenträger 23 um eine Durchgangsöffnung 81 handeln, durch welche die von dem ersten, nasalen Elektrodenträger 22 kommende Stimulationselektrode 21 zum Gewichtskörper 24 hin durchgeführt ist. Wie in der Draufsicht in Figur 12 gezeigt kann eine Verjüngung 85 der Durchgangsöffnung vorgesehen sein. Die Verjüngung 85 kann dazu eingerichtet sein, eine Führung der Stimulationselektrode 21 durch die Durchgangsöffnung zu verbessern bzw. falls erforderlich zu hemmen. Bei dem in Figur 11 gezeigten Beispiel ist es hingegen nicht erforderlich, die Stimulationselektrode 21 vollständig durch den Elektrodenträger 22 hindurchzuführen.

Ein magnetisierbares Metallelement 82 am Boden 86 des Trichters 84 dient in dem gezeigten Ausführungsbeispiel sowohl dazu einen elektrischen Kontakt zur Stimulationselektrode 21 bereitzustellen als auch der magnetischen Befestigung an dem Elektrodenhalter. Es versteht sich, dass die Elektrodenträger 22, 23 statt magnetisierbaren Metallelementen 82 auch Magnete, insbesondere elektrisch leitende Magnete aufweisen können.

Fig. 7 zeigt eine schematische Schnittdarstellung einer Stimulationselektrodenanordnung 20, welche magnetisch am Elektrodenhalter 30 befestigt ist. Hierbei ist der erste Elektrodenträger 22 am ersten Elektrodenhalter 32 magnetisch befestigt. Der zweite Elektrodenträger 23 ist am zweiten Elektrodenhalter 33 magnetisch befestigt. Der erste Elektrodenträger 22 ist ausgebildet, wie in Fig. 11 dargestellt. Der zweite Elektrodenträger 23 ist ausgebildet, wie in Fig. 12 dargestellt. Der erste Elektrodenhalter 32 weist einen ersten Magnet 34 auf, welcher das magnetisierbare Metallelement 82 am Boden 86 des Trichters 84 (vgl. Fig. 11) des ersten Elektrodenträgers 22 anzieht und den ersten Elektrodenträger 22 mittels Magnetkraft befestigt. Der zweite Elektrodenhalter 33 weist einen zweiten Magnet 35 auf, welcher das magnetisierbare Metallelement 82 am Boden 86 des Trichters 84 (vgl. Fig. 12) des zweiten Elektrodenträgers 22 anzieht und den Elektrodenträger 22 mittels Magnetkraft befestigt.

Eine elektrische Verbindung zwischen der Stimulationselektrode 21 und dem Stimulationsgerät 38 ist in Figur 7 ebenfalls schematisch dargestellt. Eine elektrische Signalübertragung zur Stimulationselektrode 21 erfolgt über eine Leitung 37, den ersten Magneten 34 des ersten Elektrodenhalters 32 und das magnetisierbare Metallelement 82 des ersten Elektrodenträgers 22. Es versteht sich, dass die Leitung 37 nur schematisch dargestellt ist und auch eine anderweitige elektrische Verbindung, beispielsweise über leitende Elemente des ersten Elektrodenhalters 32 bereitgestellt werden kann.

Der erste und/oder zweite Elektrodenhalter 32, 33 können längenverstellbar ausgebildet sein. Beispielsweise kann ein Innenteil 72 gegenüber einer äußeren Hülse 71 verschoben werden. Hierdurch kann ein Abstand des Elektrodenhalters 32, 33 zum Auge des Nutzers justiert werden. Ein Abstand zwischen dem ersten und zweiten Elektrodenhalter 32, 33 ist hingegen vorzugweise fest. Der feste Abstand ermöglicht es, die Stimulationselektrodenanordnung in einer Aufnahmeschale in einem definierten Abstand bereitzustellen und aus dieser zu entnehmen. Der erste und zweite Elektrodenträger sind dabei vorzugsweise in demselben Abstand voneinander angeordnet, wie der Abstand zwischen dem ersten und zweiten Elektrodenhalter 32,33.

Fig. 8 bis 10 zeigen den Ablauf zum Befestigen einer Stimulationselektrodenanordnung 20 an einem Elektrodenhalter 30 in perspektivischen Darstellungen.

Dabei zeigt Fig. 8 eine schematische perspektivische Darstellung des Elektrodenhalters 30 mit einem nasalen ersten Elektrodenhalter 32 und einem temporalen zweiten Elektrodenhalter 33. Der nasale Elektrodenhalter 32 weist eine stiftförmige Aufnahme mit einem ersten Magnet 34 auf. Der temporale Elektrodenhalter 33 weist eine stiftförmige Aufnahme mit einem zweiten Magnet 35 auf. Die Aufnahmen sind jeweils oval ausgebildet. Hierdurch können die Elektrodenträger 22, 23 in einer definierten Ausrichtung an den Elektrodenhaltern 32, 33 befestigt werden.

Wie in Fig. 9 gezeigt, kann im nächsten Schritt das Set 50 mit der Aufnahmeschale 40 und der darin angeordneten Stimulationselektrodenanordnung 20 an dem Elektrodenhalter 30 platziert werden, sodass die Stimulationselektrodenanordnung 20 mittels Magnetkraft aus der Aufnahmeschale 40 entnommen werden kann. Der Nutzer muss die Stimulationselektrode also nicht direkt anfassen. Dies erleichtert die Handhabung für einen sehbehinderten Nutzer. Darüber hinaus wird die Hygiene bei der Handhabung verbessert.

Fig. 10 zeigt als Ergebnis eine schematische perspektivische Darstellung des Elektrodenhalters 30 mit einer daran magnetisch befestigten Stimulationselektrodenanordnung 20.

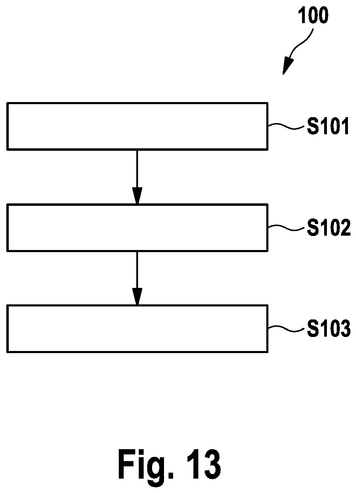

Fig. 13 zeigt ein Flussdiagramm eines Verfahrens 100 zum Befestigen einer Stimulationselektrodenanordnung an einer Vorrichtung zur Elektrostimulation des Auges.

In einem ersten Schritt S101 werden eine einer Vorrichtung zur Elektrostimulation eines Auges mit einem Elektrodenhalter 30, wie hierin offenbart, und eine Stimulationselektrodenanordnung, wie hierin offenbart, bereitgestellt.

In deinem zweiten Schritt S102 werden der erste und/oder zweite Elektrodenträger der Stimulationselektrodenanordnung und der Elektrodenhalters der Vorrichtung aneinander platziert bzw. aneinander angeordnet.

In einem dritten Schritt S103 werden der erste und/oder zweite Elektrodenträger der Stimulationselektrodenanordnung magnetisch am Elektrodenhalter der Vorrichtung befestigt.

Zusammenfasend kann gemäß Aspekten der vorliegenden Offenbarung eine Handhabung einer Stimulationselektrode zur Elektrostimulation des Auges insbesondere für sehbehinderte Nutzer weiter verbessert werden.

## Patentansprüche

1. Stimulationselektrodenanordnung (20) für eine Vorrichtung (10) zur Elektrostimulation des Auges, wobei die Vorrichtung (10) ein brillenartiges Gestell (11) mit einem Nasenteil (12), eine mit dem Nasenteil (12) verbundene Anordnung zum Halten des Gestells an dem Kopf des Patienten sowie zumindest einen an dem Gestell (11) angeordneten Elektrodenhalter (30) für eine insbesondere drahtförmige Stimulationselektrode (21) aufweist, wobei die Stimulationselektrodenanordnung (20) einen ersten Elektrodenträger (22) und einen zweiten Elektrodenträger (23) aufweist, wobei die Stimulationselektrode (21) zwischen dem ersten Elektrodenträger (22) und dem zweiten Elektrodenträger (23) angeordnet ist, wobei der erste und/oder zweite Elektrodenträger dazu eingerichtet ist, magnetisch an dem Elektrodenhalter (30) befestigt zu werden.

2. Stimulationselektrodenanordnung nach Anspruch 1, wobei der erste Elektrodenträger (22) dazu eingerichtet ist, magnetisch an einem ersten Elektrodenhalter (32) befestigt zu werden und wobei der zweite Elektrodenträger (23) dazu eingerichtet ist, magnetisch an einem zweiten Elektrodenhalter (33) befestigt zu werden.

3. Stimulationselektrodenanordnung nach einem der vorhergehenden Ansprüche, wobei der erste Elektrodenträger (22) ein nasaler Elektrodenträger ist und dazu eingerichtet ist, magnetisch an einer nasalen Aufnahme (34) des Elektrodenhalters (32) befestigt zu werden; und wobei der zweite Elektrodenträger (23) ein temporaler Elektrodenträger ist und dazu eingerichtet ist, magnetisch an einer temporalen Aufnahme (35) des Elektrodenhalters (33) befestigt zu werden.

4. Stimulationselektrodenanordnung nach einem der vorhergehenden Ansprüche, wobei der erste und/oder zweite Elektrodenträger (22, 23) einen Magnet aufweist und dazu eingerichtet ist, mittels des Magneten an dem Elektrodenhalter (30, 32, 33) befestigt zu werden.

5. Stimulationselektrodenanordnung nach einem der vorhergehenden Ansprüche, wobei der mindestens eine Elektrodenhalter (30, 32, 33) einen Magnet aufweist; und der Elektrodenträger (22, 23) dazu eingerichtet ist mittels des Magneten an dem Elektrodenhalter (30, 32, 33) befestigt zu werden; insbesondere wobei der erste und/oder zweite Elektrodenträger (22, 23) ein magnetisierbares Metall aufweist und dazu eingerichtet ist, mittels des Magneten an dem Elektrodenhalter (30, 32, 33) befestigt zu werden.

6. Stimulationselektrodenanordnung nach einem der vorhergehenden Ansprüche, wobei der erste und/oder zweite Elektrodenträger (22, 23) dazu ausgebildet ist, selbstausrichtend an dem Elektrodenhalter (30, 32, 33) befestigt zu werden, wobei der erste und/oder zweite Elektrodenträger (22, 23) einen Trichter (84) aufweist und dazu eingerichtet ist, mittels des Trichters (84) selbstausrichtend an dem Elektrodenhalter (32, 33) befestigt zu werden.

7. Stimulationselektrodenanordnung nach Anspruch 6, wobei an einem Boden (86) des Trichters (84) ein elektrischer Kontakt angeordnet ist, welcher elektrisch leitend mit der Stimulationselektrode (21) verbunden ist, insbesondere wobei ein magnetisierbares Metallelement (82) am Boden (86) des Trichters (84) angeordnet ist, wobei das magnetisierbare Metallelement (82) dazu eingerichtet ist, sowohl einen elektrischen Kontakt zur Stimulationselektrode (21) bereitzustellen als auch magnetisch an dem Elektrodenhalter (30, 32, 33) befestigt zu werden.

8. Stimulationselektrodenanordnung nach einem der Ansprüche 6 bis 7, wobei der erste und/oder zweite Elektrodenträger (22, 23) dazu eingerichtet ist, in einer definierten Ausrichtung am Elektrodenhalter (30, 32, 33) befestigt zu werden, insbesondere wobei der mindestens eine Elektrodenhalter eine ovale Aufnahme für den ersten und/oder zweiten Elektrodenträger (22, 23) aufweist.

9. Stimulationselektrodenanordnung nach einem der vorhergehenden Ansprüche, wobei die Stimulationselektrode (21) eine mit mindestens einem drahtförmigen Anschluss versehene Kontaktlinse mit Elektrodenstrukturen, insbesondere mit Elektrodenstrukturen aus einem Edelmetall, ist.

10. Stimulationselektrodenanordnung nach einem der vorhergehenden Ansprüche, wobei die Stimulationselektrode (21) mindestens an ihrem ersten Ende elektrisch leitend mit dem ersten Elektrodenträger (22) verbunden ist; und wobei der Elektrodenträger dazu eingerichtet ist, elektrisch leitend mit dem Elektrodenhalter (32) verbunden zu werden.

11. Set (50) mit einer Stimulationselektrodenanordnung (20) nach einem der vorhergehenden Ansprüche und einer Aufnahmeschale (40), wobei der erste Elektrodenträger (22) und der zweite Elektrodenträger (23) derart in der Aufnahmeschale (40) angeordnet und dazu eingerichtet sind, dass sie mittels einer zwischen einem Elektrodenhalter (30, 32, 33) und dem ersten und/oder zweiten Elektrodenträger (22, 23) wirkenden Magnetkraft aus der Aufnahmeschale (40) entnehmbar sind.

12. Set nach Anspruch 11, wobei der erste Elektrodenträger (22) und der zweite Elektrodenträger (23) mit einer Haltekraft klemmend in der Aufnahmeschale (40) befestigt sind und wobei die Magnetkraft größer ist als die Haltekraft, so dass der ersten und/oder zweiten Elektrodenträger (22, 23) mittels der Magnetkraft aus der Aufnahmeschale (40) entnehmbar sind.

13. Vorrichtung (10) zur Elektrostimulation des Auges, mit einem brillenartigen Gestell (11) mit einem Nasenteil (12), eine mit dem Nasenteil (12) verbundene Anordnung zum Halten des Gestells an dem Kopf des Patienten sowie zumindest einen an dem Gestell (11) angeordneten Elektrodenhalter (30) für eine Stimulationselektrodenanordnung (20) mit einer insbesondere drahtförmigen Stimulationselektrode (21), wobei die Stimulationselektrodenanordnung (20) einen ersten Elektrodenträger (22) und einen zweiten Elektrodenträger (23) aufweist, wobei die Stimulationselektrode (21) zwischen dem ersten Elektrodenträger (22) und dem zweiten Elektrodenträger (23) angeordnet ist, wobei der Elektrodenhalter (30) dazu eingerichtet ist, den erste und/oder zweite Elektrodenträger (22, 23) magnetisch an dem Elektrodenhalter (30) zu befestigen.

14. System (1) zur Elektrostimulation des Auges mit einer Stimulationselektrodenanordnung (20) nach einem der Ansprüche 1 bis 10 und einer korrespondierenden Vorrichtung (10) nach Anspruch 13.

15. Verfahren zum Befestigen einer Stimulationselektrodenanordnung (20) an einer Vorrichtung (10) zur Elektrostimulation des Auges, wobei das Verfahren folgende Schritte aufweist:
a) Bereitstellen einer Vorrichtung (10) zur Elektrostimulation eines Auges nach Anspruch 13 und einer Stimulationselektrodenanordnung (20) nach einem der Ansprüche 1 bis 10;
b) Platzieren des ersten und/oder zweiten Elektrodenträgers (22, 23) des Stimulationselektrodenanordnung (20) und des Elektrodenhalters (30, 32, 33) der Vorrichtung (10) aneinander; und
c) magnetisches Befestigen des ersten und/oder zweiten Elektrodenträgers (22, 23) der Stimulationselektrodenanordnung (20) an dem Elektrodenhalter (30, 32, 33) der Vorrichtung (10).

## Claims

1. Stimulation electrode arrangement (20) for a device (10) for electrostimulation of the eye, wherein the device (10) comprises a spectacle-like frame (11) with a nose part (12), an arrangement connected to the nose part (12) for holding the frame on the head of the patient, and at least one electrode holder (30) arranged at the frame (11) for a stimulation electrode (21), in particular a wire-shaped stimulation electrode, wherein the stimulation electrode arrangement (20) comprises a first electrode carrier (22) and a second electrode carrier (23), wherein the stimulation electrode (21) is arranged between the first electrode carrier (22) and the second electrode carrier (23), wherein the first and/or second electrode carrier is adapted to be magnetically attached to the electrode holder (30).

2. Stimulation electrode arrangement according to claim 1, wherein the first electrode carrier (22) is adapted to be magnetically attached to a first electrode holder (32) and wherein the second electrode carrier (23) is adapted to be magnetically attached to a second electrode holder (33).

3. Stimulation electrode arrangement according to any of the preceding claims, wherein the first electrode carrier (22) is a nasal electrode carrier and is adapted to be magnetically attached to a nasal receptacle (34) of the electrode holder (32); and wherein the second electrode carrier (23) is a temporal electrode carrier and is adapted to be magnetically attached to a temporal receptacle (35) of the electrode holder (33).

4. Stimulation electrode arrangement according to any of the preceding claims, wherein the first and/or second electrode carrier (22, 23) comprises a magnet and is adapted to be attached to the electrode holder (30, 32, 33) by the magnet.

5. Stimulation electrode arrangement according to any of the preceding claims, wherein the at least one electrode holder (30, 32, 33) comprises a magnet; and the electrode carrier (22, 23) is adapted to be attached to the electrode holder (30, 32, 33) by the magnet; in particular wherein the first and/or second electrode carrier (22, 23) comprises a magnetizable metal and is adapted to be attached to the electrode holder (30, 32, 33) by the magnet.

6. Stimulation electrode arrangement according to any of the preceding claims, wherein the first and/or second electrode carrier (22, 23) is adapted to be attached self-aligning at the electrode holder (30, 32, 33), wherein the first and/or second electrode carrier (22, 23) comprises a funnel (84) and is adapted to be attached in a self-aligning manner at the electrode holder (32, 33) by the funnel (84).

7. Stimulation electrode arrangement according to claim 6, wherein an electrical contact is arranged on a bottom (86) of the funnel (84), which is electrically conductively connected to the stimulation electrode (21), in particular wherein a magnetizable metal element (82) is arranged on the bottom (86) of the funnel (84), wherein the magnetizable metal element (82) is adapted to provide both an electrical contact to the stimulation electrode (21) and to be magnetically attached at the electrode holder (30, 32, 33).

8. Stimulation electrode arrangement according to any of claims 6 to 7, wherein the first and/or second electrode carrier (22, 23) is adapted to be attached in a defined orientation at the electrode holder (30, 32, 33), in particular wherein the at least one electrode holder comprises an oval receptacle for the first and/or second electrode carrier (22, 23).

9. Stimulation electrode arrangement according to any of the preceding claims, wherein the stimulation electrode (21) is a contact lens provided with at least one wire-shaped connection and with electrode structures, in particular with electrode structures made of a noble metal.

10. Stimulation electrode arrangement according to any of the preceding claims, wherein the stimulation electrode (21) is at least at its first end electrically conductively connected to the first electrode carrier (22); and wherein the electrode carrier is adapted to be electrically conductively connected to the electrode holder (32).

11. Set (50) with a stimulation electrode arrangement (20) according to any of the preceding claims and a holding tray (40), wherein the first electrode carrier (22) and the second electrode carrier (23) are arranged in the receiving shell (40) and are adapted to be taken out of the holding tray (40) by a magnetic force acting between an electrode holder (22) and the first and/or second electrode carrier (23).

12. Set according to claim 11, wherein the first electrode carrier (22) and the second electrode carrier (23) are mounted in the receiving shell (40) clamped with a holding force and wherein the magnetic force is greater than the holding force, such that the first and/or second electrode carriers (22, 23) can be taken out of the receiving shell (40) by the magnetic force.

13. Device (10) for electrostimulation of the eye, with a spectacle-like frame (11) with a nose part (12), an arrangement connected to the nose part (12) for holding the frame on the head of the patient, and at least one electrode holder (30) arranged at the frame (11) for a stimulation electrode arrangement (20) with a stimulation electrode (21), in particular a wire-shaped stimulation electrode, wherein the stimulation electrode arrangement (20) comprises a first electrode carrier (22) and a second electrode carrier (23), wherein the stimulation electrode (21) is arranged between the first electrode carrier (22) and the second electrode carrier (23), wherein the electrode holder (30) is adapted to magnetically attach the first and/or second electrode carrier (22, 23) to the electrode holder (30).

14. System (1) for electrostimulation of the eye with a stimulation electrode arrangement (20) according to any of claims 1 to 10 and a corresponding device (10) according to claim 13.

15. Method for attaching a stimulation electrode arrangement (20) to a device (10) for electrostimulation of the eye, wherein the method comprises the following steps:
a) providing a device (10) for electrostimulation of an eye according to claim 13 and a stimulation electrode arrangement (20) according to any of claims 1 to 10;
b) placing the first and/or second electrode carriers (22, 23) of the stimulation electrode arrangement (20) and the electrode holder (30, 32, 33) of the device (10) at each other; and
c) magnetically attaching the first and/or second electrode carriers (22, 23) of the stimulation electrode arrangement (20) to the electrode holder (30, 32, 33) of the device (10).

## Revendications

1. Agencement d'électrode de stimulation (20) pour un dispositif (10) permettant l'électrostimulation de l'œil, dans lequel le dispositif (10) présente une monture (11) en forme de lunettes comportant une partie nasale (12), un agencement relié à la partie nasale (12) permettant de retenir la monture sur la tête du patient ainsi qu'au moins un porte-électrode (30) disposé sur la monture (11) pour une électrode de stimulation (21) en particulier en forme de fil, dans lequel l'agencement d'électrode de stimulation (20) présente un premier support d'électrode (22) et un second support d'électrode (23), dans lequel l'électrode de stimulation (21) est disposée entre le premier support d'électrode (22) et le second support d'électrode (23), dans lequel le premier et/ou le second support d'électrode sont conçus pour être fixés magnétiquement au porte-électrode (30).

2. Agencement d'électrode de stimulation selon la revendication 1, dans lequel le premier support d'électrode (22) est conçu pour être fixé magnétiquement à un premier porte-électrode (32), et dans lequel le second support d'électrode (23) est conçu pour être fixé magnétiquement à un second porte-électrode (33).

3. Agencement d'électrode de stimulation selon l'une des revendications précédentes, dans lequel le premier support d'électrode (22) est un support d'électrode nasal et est conçu pour être fixé magnétiquement à un logement nasal (34) du porte-électrode (32) ; et dans lequel le second support d'électrode (23) est un support d'électrode temporal et est conçu pour être fixé magnétiquement à un logement temporal (35) du porte-électrode (33).

4. Agencement d'électrode de stimulation selon l'une des revendications précédentes, dans lequel le premier et/ou le second support d'électrode (22, 23) présentent un aimant et sont conçus pour être fixés au porte-électrode (30, 32, 33) au moyen de l'aimant.

5. Agencement d'électrode de stimulation selon l'une des revendications précédentes, dans lequel l'au moins un porte-électrode (30, 32, 33) présente un aimant ; et le support d'électrode (22, 23) est conçu pour être fixé au porte-électrode (30, 32, 33) au moyen de l'aimant ; en particulier dans lequel le premier et/ou le second support d'électrode (22, 23) présentent un métal magnétisable et sont conçus pour être fixés au porte-électrode (30, 32, 33) au moyen de l'aimant.

6. Agencement d'électrode de stimulation selon l'une des revendications précédentes, dans lequel le premier et/ou le second support d'électrode (22, 23) sont conçus pour être fixés au porte-électrode (30, 32, 33) de manière à être autoalignés, dans lequel le premier et/ou le second support d'électrode (22, 23) présentent un entonnoir (84) et sont conçus pour être fixés au porte-électrode (32, 33) de manière à être autoalignés au moyen de l'entonnoir (84).

7. Agencement d'électrode de stimulation selon la revendication 6, dans lequel un contact électrique est disposé sur un fond (86) de l'entonnoir (84), lequel contact électrique est connecté de manière électriquement conductrice à l'électrode de stimulation (21), en particulier dans lequel un élément métallique magnétisable (82) est disposé sur le fond (86) de l'entonnoir (84), dans lequel l'élément métallique magnétisable (82) est configuré à la fois pour fournir un contact électrique à l'électrode de stimulation (21) et pour être fixé magnétiquement au porte-électrode (30, 32, 33).

8. Agencement d'électrode de stimulation selon l'une des revendications 6 à 7, dans lequel le premier et/ou le second support d'électrode (22, 23) sont conçus pour être fixés au porte-électrode (30, 32, 33) selon un alignement défini, en particulier dans lequel l'au moins un porte-électrode présente un logement ovale pour le premier et/ou le second support d'électrode (22, 23).

9. Agencement d'électrode de stimulation selon l'une des revendications précédentes, dans lequel l'électrode de stimulation (21) est une lentille de contact pourvue d'au moins une connexion en forme de fil et comportant des structures d'électrode, en particulier comportant des structures d'électrode en un métal précieux.

10. Agencement d'électrode de stimulation selon l'une des revendications précédentes, dans lequel l'électrode de stimulation (21) est connectée de manière électriquement conductrice, au moins au niveau de sa première extrémité, au premier support d'électrode (22) ; et dans lequel le support d'électrode est configuré pour être connecté de manière électriquement conductrice au porte-électrode (32).

11. Ensemble (50) comportant un agencement d'électrode de stimulation (20) selon l'une des revendications précédentes et un plateau de réception (40), dans lequel le premier support d'électrode (22) et le second support d'électrode (23) sont disposés dans le plateau de réception (40) et sont conçus de telle sorte qu'ils peuvent être retirés du plateau de réception (40) au moyen d'une force magnétique agissant entre un porte-électrode (30, 32, 33) et le premier et/ou le second support d'électrode (22, 23).

12. Ensemble selon la revendication 11, dans lequel le premier support d'électrode (22) et le second support d'électrode (23) sont fixés par serrage dans le plateau de réception (40) avec une force de retenue et dans lequel la force magnétique est supérieure à la force de retenue de sorte que le premier et/ou le second support d'électrode (22, 23) peuvent être retirés du plateau de réception (40) au moyen de la force magnétique.

13. Dispositif (10) permettant l'électrostimulation de l'œil, comportant une monture (11) en forme de lunettes comportant une partie nasale (12), un agencement relié à la partie nasale (12) permettant de retenir la monture sur la tête du patient ainsi qu'au moins un porte-électrode (30) disposé sur la monture (11) pour un agencement d'électrode de stimulation (20) comportant une électrode de stimulation (21) en particulier en forme de fil, dans lequel l'agencement d'électrode de stimulation (20) présente un premier support d'électrode (22) et un second support d'électrode (23), dans lequel l'électrode de stimulation (21) est disposée entre le premier support d'électrode (22) et le second support d'électrode (23), dans lequel le porte-électrode (30) est conçu pour fixer magnétiquement le premier et/ou le second support d'électrode (22, 23) au porte-électrode (30).

14. Système (1) permettant l'électrostimulation de l'œil comportant un agencement d'électrode de stimulation (20) selon l'une des revendications 1 à 10 et un dispositif (10) correspondant selon la revendication 13.

15. Procédé permettant la fixation d'un agencement d'électrode de stimulation (20) à un dispositif (10) permettant l'électrostimulation de l'œil, dans lequel le procédé présente les étapes suivantes :
a) fourniture d'un dispositif (10) permettant l'électrostimulation d'un œil selon la revendication 13 et d'un agencement d'électrode de stimulation (20) selon l'une des revendications 1 à 10 ;
b) placement du premier et/ou du second support d'électrode (22, 23) de l'agencement d'électrode de stimulation (20) et du porte-électrode (30, 32, 33) du dispositif (10) les uns contre les autres ; et
c) fixation magnétique du premier et/ou du second support d'électrode (22, 23) de l'agencement d'électrode de stimulation (20) au porte-électrode (30, 32, 33) du dispositif (10).
